# EUROPEAN PATENT APPLICATION

(11) **EP 4 151 168 A2**
(43) Date of publication of application: **22.03.2023**
(21) Application number: 22196121.2
(22) Date of filing: 16.09.2022
(51) Int. Cl.: A61B 18/12, A61B 18/14, A61B 34/20, A61B 34/10, A61B 18/00, A61B 90/00

(54) **ABLATING A REGION OF PATIENT ORGAN USING SELECTED ABLATION ELECTRODES OF AN EXPANDABLE CATHETER**

(30) Priority: 20.09.2021 US 202117479272
(71) Applicant: Biosense Webster (Israel) Ltd., Yokneam 2066717 (IL)
(72) Inventor: KORGOZKI, Yan, 2066717 Yokneam (IL); GLINER, Vadim, 2066717 Yokneam (IL); GOLDENBERG, Ilan, 2066717 Yokneam (IL)
(74) Representative: Carpmaels & Ransford LLP

(57) **Abstract**

A method includes receiving: (i) a position of a target tissue intended to be ablated in an organ of a patient and having a predefined pattern, and (ii) an energy level of an ablation signal intended to be applied to the target tissue. One or more selected ablation electrodes that, when applying the ablation signal, produce together a lesion having a shape that covers the predefined pattern, are selected in a catheter that is inserted into the organ and has an array of ablation electrodes. In response to verifying that: (i) the one or more selected ablation electrodes are positioned on the target tissue, and (ii) a contact force between the one or more selected ablation electrode and the target tissue is larger than a force threshold, the ablation signal is applied to the target tissue using the one or more selected ablation electrodes.

## Description

### FIELD OF THE INVENTION

The present invention relates generally to medical devices, and particularly to methods and systems for ablating tissue using selected ablation electrodes of an expandable catheter.

### BACKGROUND OF THE INVENTION

Various techniques for ablating tissue of a patient organ using selected ablation electrodes have been published.

For example, U.S. Patent Application Publication No. 2020/0146743 describes a spinal tumor ablation devices and related systems and methods. Some spinal tumor ablation devices include two conductors and one or more thermocouples. The one or more thermocouples are utilized to measure a temperature at a location on one of the conductors. A generator can produce an electrical alternating current to be conducted between the first conductor and the second conductor via tissue within a desired ablation region. A processor may monitor temperature and impedance and control an output of the generator when the impedance of the tissue increases and stop the generator when the thermal energy delivered to the tissue reaches a target threshold.

U.S. Patent Application Publication No. 2017/0128119 describes a prediction of atrial wall electrical reconnection based on contact force measured during RF ablation.

### SUMMARY OF THE INVENTION

An embodiment of the present invention that is described herein provides a method including receiving: (i) a position of a target tissue intended to be ablated in an organ of a patient and having a predefined pattern, and (ii) an energy level of an ablation signal intended to be applied to the target tissue. One or more selected ablation electrodes that, when applying the ablation signal, produce together a lesion having a shape that covers the predefined pattern, are selected in a catheter that is inserted into the organ and has an array of ablation electrodes. In response to verifying that the one or more selected ablation electrodes are positioned on the target tissue, the ablation signal is applied to the target tissue using the one or more selected ablation electrodes.

In some embodiments, applying the ablation signal to the target tissue includes monitoring a cumulative energy of the ablation signal applied to the target tissue, and in response to detecting that the cumulative energy exceeds the energy level, terminating the ablation signal to the one or more selected ablation electrodes. In other embodiments, monitoring the cumulative energy includes monitoring an ablation power of the ablation signal and a time interval of applying the ablation signal to the target tissue using the one or more selected ablation electrodes. In yet other embodiments, the target tissue includes a first section at a first position and a second section at a second position different from the first position, the method includes receiving a position signal indicative of an additional position of the array of ablation electrodes, and calculating electrodes positions of the ablation electrodes of the array, respectively, and the one or more selected ablation electrodes include at least a first ablation electrode for producing a first lesion that covers the first section and a second ablation electrode for producing a second lesion that that covers the second section.

In an embodiment, the method includes verifying that a contact force between the one or more selected ablation electrode and the target tissue is larger than a force threshold, applying the ablation signal includes verifying that the first ablation electrode is positioned on the first section and the second ablation electrode is positioned on the second section, and subsequently, verifying that the contact force between: (i) the first ablation electrode and the first section, and (ii) the second ablation electrode and the second section, is larger than the force threshold. In another embodiment, when applying the ablation signal, in response to detecting that at least one of: (i) at least the first electrode is moved relative to the first section, and (ii) the contact force between at least the first ablation electrode and the first section is smaller than the force threshold, terminating the ablation signal to at least the first ablation electrode. In yet another embodiment, receiving the first and second positions includes receiving a region indicative of the target tissue, and receiving a first coordinate defining the first section, and a second coordinate defining the second section, and selecting the first and second electrodes includes selecting: (i) the first ablation electrode that falls on the first coordinate and (ii) the second ablation electrode that falls on the second coordinate.

In some embodiments, receiving the energy level includes receiving a first energy level of a first ablation signal intended to be applied to the first section, and a second energy level of a second ablation signal intended to be applied to the second section, and defining the first and second sections is based on at least one of: (i) a geometrical shape of the first and second sections, and (ii) the first and second energy levels. In other embodiments, the first energy level differs from the second energy level. In yet other embodiments, the catheter includes an expandable distal-end assembly having the first and second ablation electrode and a third ablation electrode not selected by the processor, and the ablation signal is not applied to the third ablation electrode.

There is additionally provided, in accordance with an embodiment of the present invention, a system including an interface and a processor. The interface is configured to receive: (i) a position of a target tissue intended to be ablated in an organ of a patient and having a predefined pattern, and (ii) an energy level of an ablation signal intended to be applied to the target tissue. The processor is configured to: (a) select, in a catheter that is inserted into the organ and having an array of ablation electrodes, one or more selected ablation electrodes that, when applying the ablation signal, produce together a lesion having a shape that covers the predefined pattern, and (b) in response to verifying that the one or more selected ablation electrodes are positioned on the target tissue, control a generator to apply the ablation signal to the target tissue using the one or more selected ablation electrodes.

### BRIEF DESCRIPTION OF THE DRAWINGS

The present invention will be more fully understood from the following detailed description of the embodiments thereof, taken together with the drawings in which:
Fig. 1 is a schematic, pictorial illustration of a catheter-based tracking and ablation system, in accordance with an exemplary embodiment of the present invention;
Fig. 2A is a pictorial illustration of a region of a heart of a patient in accordance with the present invention.
Fig. 2B is a schematic, pictorial illustration of a distal-end assembly of a catheter intended to apply ablation signals to an ablation region in a patient heart, in accordance with an exemplary embodiment of the present invention; and
Fig. 3 is a flow chart that schematically illustrates a method for applying ablation signals to tissue using some of the electrodes of the catheter of Fig. 2, in accordance with an exemplary embodiment of the present invention.

### DETAILED DESCRIPTION OF EMBODIMENTS

### OVERVIEW

Catheters having expandable distal end assemblies, such as a balloon catheter or a basket catheter, may be used for ablating tissue in a patient organ, such as a patient heart. Such expandable catheters typically have ablation electrodes disposed on and/or around the outer surface of the balloon or splines of the basket. Thus, balloon catheters or basket catheters may be used for ablating an annular section of an organ, such as an ostium of a pulmonary vein (PV) of the patient heart, so as to obtain a lesion having a shape of a full circle (e.g., a perimeter) along the annular section.

In some cases, an ablation procedure may require ablating only a partial section of the ostium, or ablation of any other target tissue have a non-tubular shape. In such procedures, it is important to have sufficiently high contact between the ablation electrodes that are intended to apply the ablation signals, and the target tissue, e.g., the partial section or the tissue having the non-tubular shape.

Moreover, in such procedures, a physician that performs the ablation procedure needs to select the correct ablation electrodes of the catheter, and to monitor the selected electrodes, so as to verify that each electrode is in a sufficiently high contact with the tissue while applying ablation signals to the target tissue.

Embodiments of the present invention that are described hereinbelow provide improved techniques for using an expandable catheter to apply ablation signals to a partial section of a tubular organ, or for ablating any other target tissue having a non-tubular shape.

In some embodiments, a system for performing ablation to tissue comprises a catheter having an expandable distal-end assembly, such as a balloon catheter. The catheter has multiple ablation electrodes coupled to an outer surface of the balloon, and typically but not necessarily are arranged uniformly on the outer surface. The ablation electrodes are configured to be placed in contact with a target tissue, and to apply one or more ablation signals to the tissue in question. When the catheter is in an expanded position, the ablation electrodes are pressed against the tissue intended to be ablated, and therefore, a sufficiently high contact force is applied between the ablation electrodes and the tissue. In some cases, however, only a non-annular section of the PV needs to be ablated, or the target tissue may have a non-tubular shape.

In some embodiments, the system comprises an interface, which is configured to receive one or more positions (e.g., coordinates) of one or more sections of the target tissue intended to be ablated, and one or more energy levels intended to be applied, respectively, to one or more respective sections of the target tissue. For example, a first section may require a first energy level and a second section may require a second energy level, which is different from the first energy level.

In some embodiments, the system comprises a processor, which is configured to select, from among an array of the ablation electrodes, one or more ablation electrodes that fall on the one or more sections. For example, the target tissue intended to be ablated comprises first and second sections, and the processor is configured to select at least first and second electrodes that fall on the positions of the first and second sections, respectively. Note that one or more electrodes of the array are not selected by the processor.

In some embodiments, the processor is configured to check whether or not the first and second ablation electrodes are positioned on the first and second sections, respectively. The processor is further configured to hold a force threshold, which is indicative of the required contact force applied between the first and second ablation electrode and the first and second respective sections of the tissue intended to be ablated. In the present example, the threshold is indicative of the required minimal contact force. In other words, when the applied contact force is smaller than the threshold, the ablation may fail to produce a lesion that has the required properties for blocking the propagation of an electrophysiological wave through the target tissue.

In some embodiments, the processor is configured to check, for each of the first and second ablation electrodes that are intended to apply the ablation signals, whether or not the contact force between the first and second ablation electrode and the tissue of the respective first and second sections is larger than the force threshold.

In some embodiments, the processor is configured to control a radiofrequency (RF) generator to apply one or more ablation signals to the first and second respective sections, in response to verifying that: (i) at least one of and typically each of the first and second ablation electrodes are positioned on the respective first and second sections intended to be ablated, and (ii) the contact force between the first and second ablation electrodes and the first and second sections, respectively, is larger than the force threshold.

In some embodiments, the processor is configured to select first and second ablation signals to be applied to the first and second ablation electrodes, respectively. In such embodiments, the processor is configured to control the RF generator, and/or a device that is configured to route the ablation signals to the selected first and second ablation electrodes. In such embodiments, the first ablation electrode, which is positioned accurately on the first section and has a sufficiently high contact force with the tissue, applies the first ablation signal to the first section, whereas the second electrode that is either not positioned on the second section, or applies a contact force smaller than the force threshold, may not receive the second ablation signal. In alternative embodiments, in case at least one of the first and second electrodes is not positioned on the respective section and/or is not applying a contact force larger than the force threshold, the processor will prevent both the first and second electrodes from applying the first and second ablation signals.

Note that one or more other electrodes of the array that were not selected by the processor, will not receive any ablation signal.

In some embodiments, the processor is configured to monitor the cumulative energy applied by each of the first and second ablation electrodes. In the present example, the processor controls the power of the ablation signal, and monitors the time interval (i.e., duration) in which the ablation signal has been applied to the first and second sections of the target tissue. The processor holds a first energy level corresponding to the ablation energy intended to be applied to the first section, and a second energy level corresponding to the ablation energy intended to be applied to the second section. During the ablation, the processor monitors the cumulative energy applied to the first and second sections, and once the cumulative energy applied to a given section is equal to (or slightly larger than) the respective energy level, the processor is configured to terminate the ablation signal applied to the given section.

The disclosed techniques provide a physician with the flexibility to apply ablation signals to tissue using any selected electrodes in a multi-electrode catheter, and particularly in a catheter having an expandable distal end. Moreover, the disclosed techniques enable to automatically control the quality of the lesion produced in the ablation of non-tubular organs, or in ablation of a portion of an annular section of an organ.

### SYSTEM DESCRIPTION

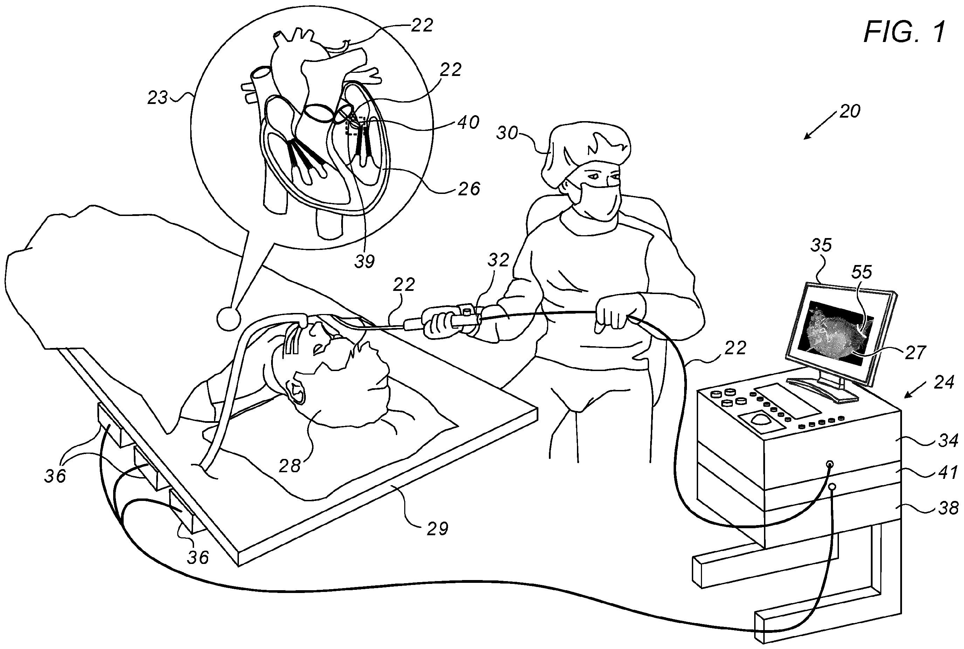

Fig. 1 is a schematic, pictorial illustration of a catheter-based tracking and ablation system 20, in accordance with an exemplary embodiment of the present invention.

In some embodiments, system 20 comprises a catheter 22, in the present example a cardiac catheter, and a control console 24. In the embodiment described herein, catheter 22 may be used for any suitable therapeutic and/or diagnostic purposes, such as sensing electro-anatomical signals and/or ablation of tissue in a heart 26.

In some embodiments, console 24 comprises a processor 34, typically a general-purpose computer, with suitable front end and interface circuits for receiving signals via catheter 22 and for controlling the other components of system 20 described herein. Console 24 further comprises a user display 35, which is configured to receive from processor 34 a map 27 of heart 26, and to display the map 27.

In some embodiments, map 27 may comprise any suitable type of three-dimensional (3D) anatomical map produced using any suitable technique. For example, the anatomical map may be produced using an anatomical image produced by using a suitable medical imaging system, or using a fast anatomical mapping (FAM) techniques available in the CARTO^{™} system, produced by Biosense Webster Inc. (Irvine, Calif.), or using any other suitable technique, or using any suitable combination of the above.

Reference is now made to an inset 23. In some embodiments, prior to performing an ablation procedure, a physician 30 inserts through the vasculature system of a patient 28 lying on a table 29, a catheter (can be catheter 22 or another catheter, not shown) having sensing electrodes, so as to perform electro-anatomical (EA) mapping of tissue in question of heart 26.

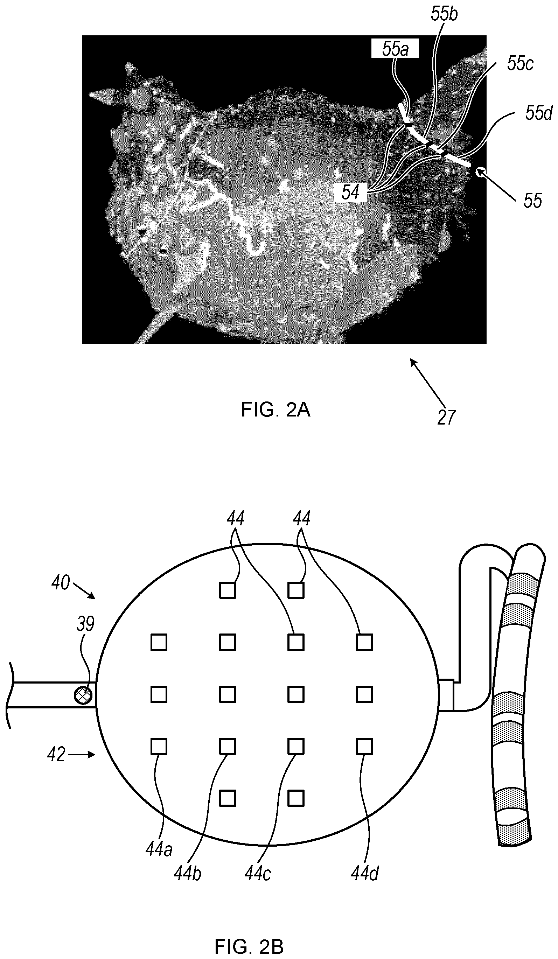

In some embodiments, catheter 22 comprises an expandable distal-end assembly 40, such as a balloon having one or more ablation electrodes for ablating a selected target tissue of heart 26. The structure of distal-end assembly 40, and the properties of the target tissue are depicted in detail in and described with respect to Figs. 2A and B below.

In some embodiments, distal-end assembly 40 comprises a position sensor, in the present example, a magnetic position sensor 39 of a magnetic position tracking system, which is configured to track the position of the distal-end assembly 40 in heart 26 or in the vasculature of patient 28. In the present example, console 24 comprises a driver circuit 41, which is configured to drive magnetic field generators 36 placed at known positions external to patient 28 lying on table 29, e.g., below the patient's torso. In the present example, position sensor 39 is coupled to distal-end assembly 40 and is configured to generate position signals in response to sensed external magnetic fields from field generators 36. The position signals are indicative of the position of distal-end assembly 40 of catheter 22 in the coordinate system of the position tracking system.

This method of position sensing is implemented in various medical applications, for example, in the CARTO^{™} system, produced by Biosense Webster Inc. (Irvine, Calif.) and is described in detail in U.S. Patent Nos. 5,391,199, 6,690,963, 6,484,118, 6,239,724, 6,618,612 and 6,332,089, in PCT Patent Publication WO 96/05768, and in U.S. Patent Application Publication Nos. 2002/0065455 A1, 2003/0120150 A1 and 2004/0068178 A1, whose disclosures are all incorporated herein by reference.

In some embodiments, the coordinate system of the position tracking system are registered with the coordinate systems of system 20 and map 27, so that processor 34 is configured to display, the position of distal-end assembly 40, over the anatomical or (EA) map (e.g., map 27).

In some embodiments, processor 34, typically comprises a general-purpose computer, which is programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

In some embodiments, the proximal end of catheter 22 is connected, *inter alia,* to interface circuits (not shown), so as to transfer: (i) the sensed signals to processor 34 for performing the EA mapping, and (ii) the ablation signals (e.g., pulses) from the ablation electrodes to the tissue of heart 26. In some embodiments, during the EA mapping, the signals produced by the ablation electrodes of distal-end assembly 40 may comprise thousands of data points, e.g., about 50,000 data points or even more, which may be stored in a memory 38 of console 24. Based on the data points, processor 34 is configured to present on map 27, wave vectors which are indicative of electrical signals propagating over the surface of heart 26.

In the context of the present disclosure and in the claims, the terms "about" or "approximately" for any numerical values or ranges indicate a suitable dimensional tolerance that allows the part or collection of components to function for its intended purpose as described herein.

In some embodiments, based on the presented wave vectors, physician 30 and/or processor 34, may identify one or more types of arrhythmias that may have occurred in heart 26. Moreover, physician 30 may determine one or more sites for treating the arrhythmias, e.g., by ablating a selected tissue in heart 26, as will be described in detail in Fig. 2 below.

In some cases, an ablation site may comprise a tubular element, such as a pulmonary vein (PV) (not shown) of heart 26, so that the balloon of distal-end assembly 40 is inserted into the PV for ablating tissue of an annular section of the PV. This ablation process is also referred to herein as a PV isolation ablation procedure.

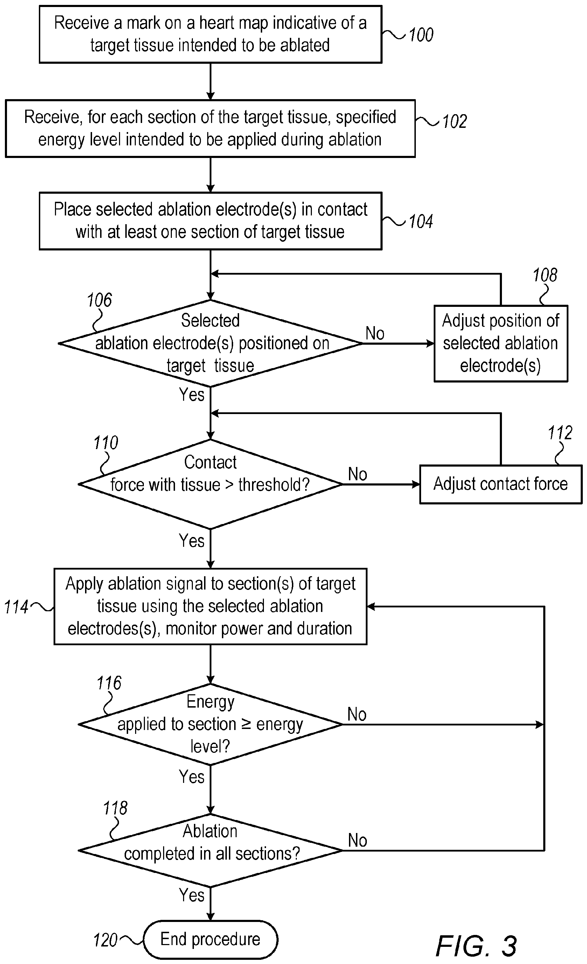

In the present example, physician 30 may define an ablation site comprising a surface of a target tissue of heart 26, referred to herein as a region 55, which is marked on map 27 by physician 30 (and/or by processor 34). Note that when the ablation site is not tubular, only a portion of the balloon is placed in contact with region 55. In some embodiments, processor 34 is configured to control the ablation pulses applied to each electrode of the balloon, using techniques described in detail in Figs. 2 and 3 below.

After determining the ablation plan, physician 30 navigates distal-end assembly 40 in close proximity to the tissue of region 55 in heart 26 e.g., using a manipulator 32 for manipulating catheter 22. Subsequently, physician 30 places one or more of the ablation electrodes in contact with the target tissue, and applies, to the tissue, one or more ablation signals.

This particular configuration of system 20 is shown by way of example, in order to illustrate certain problems that are addressed by embodiments of the present invention and to demonstrate the application of these embodiments in enhancing the performance of such a system. Embodiments of the present invention, however, are by no means limited to this specific sort of example system, and the principles described herein may similarly be applied to other sorts of medical systems.

### ABLATING A REGION USING SOME OF THE ELECTRODES OF A BALOON CATHETER

Fig. 2A is a schematic, pictorial illustration of map 27 and Fig. 2B is a schematic, pictorial illustration of the distal-end assembly 40, in accordance with an embodiment of the present invention.

Reference is now made to distal-end assembly 40. In some embodiments, distal-end assembly 40 comprises a balloon 42 having multiple ablation electrodes 44, 44a, 44b, 44c and 44d disposed on the outer surface of balloon 42. In the present example, electrodes 44, 44a, 44b, 44c and 44d are configured to be placed in contact with tissue of heart 26, and to apply one or more ablation signals, which are received from catheter 22, to the tissue of heart 26.

In some embodiments, in an expanded position, each ablation electrode of distal-end assembly 40 (e.g., electrodes 44, 44a, 44b, 44c and 44d) is located at a known position relative to position sensor 39. Based on the position signals received from position sensor 39, processor 34 is configured to estimate the positions of ablation electrodes 44, 44a, 44b, 44c and 44d in the coordinate system of map 27. For example, processor 34 may calculate a vector between position sensor 39 and a given electrode 44, and add the vector to the position of sensor 39 reported by the position tracking system, as described in Fig. 1 above.

In other embodiments, distal-end assembly 40 may have any other suitable configuration, such as but not limited to a basket catheter having multiple splines, each spline having multiple ablation electrodes mounted thereon.

Reference is now made to map 27. In some embodiments, physician 30 may define region 55, which may be marked on map 27 manually by physician 30 or automatically by processor 34. In the present example, region 55 has a shape of a line (e.g., one-dimensional), but in other embodiments, region 55 may have a two-dimensional (2D) shape or a three-dimensional (3D) shape.

In some embodiments, physician 30 may define the amount of ablation energy intended to be applied to sections (e.g., points or areas) located within region 55. In some cases, the amount of ablation energy is uniform across and/or along region 55, but in other cases, different sections of region 55 may require a different amount of ablation energy.

In some embodiments, processor 34 is configured to define sections 55a, 55b, 55c, and 55d based on: (i) the geometry (e.g., size and shape) of region 55, and (ii) the amount of ablation energy intended to be applied to each part of region 55, which is predefined by a user of system 20 (e.g., physician 30).

In some embodiments, processor 34 is configured to select, from among an array of the ablation electrodes of distal-end assembly 40, one or more ablation electrodes that fall on a predefined pattern of region 55 (that was defined by physician 30), and more specifically, on the predefined pattern of sections 55a-55d. In the present example, processor 34 is configured to select ablation electrodes 44a, 44b, 44c and 44d that fall on the pattern of sections 55a, 55b, 55c and 55d, respectively. Note that processor 34 is configured to receive the position of distal-end assembly 40 and the positions of the ablation electrodes, and based on the position of the ablation electrodes, processor 34 is configured to select the most suitable ablation electrodes. In some embodiments, when physician 30 places distal-end assembly 40 in contact with the tissue of heart 26, processor 34 is configured to identify that ablation electrodes 44a, 44b, 44c and 44d are placed in contact with the tissue intended to be ablated. In other words, when physician 30 places distal-end assembly 40 in contact with the tissue of heart 26, processor 34 is configured to automatically select ablation electrodes 44a, 44b, 44c and 44d based on position signals received from position sensor 39 and/or other position signals received from other suitable sort of position tracking systems, such as impedance-based active current location (ACL) system. One set of example implementations of a magnetic-based and an impedance-based position tracking system is described in U.S. Patent Number 7,536,218 to Govari et al., whose disclosure is incorporated herein by reference.

In such embodiments, the predefined pattern of sections 55a, 55b, 55c and 55d is fully covered by ablation electrodes 44a, 44b, 44c and 44d. Moreover, when applying the ablation signal(s) to the selected ablation electrodes, ablation electrodes 44a, 44b, 44c and 44d, are configured to produce together a lesion having a shape that covers the predefined pattern of region 55.

In some embodiments, processor 34 is configured to present virtual separators 54 between each pair of the sections of region 55, so as to assist physician in placing the selected ablation electrodes 44a-44d over sections 55a-55d, respectively. In other embodiments, processor 34 is configured to present sections 55a, 55b, 55c, and 55d on display 35 without separators 54.

In the present example, processor 34 defines the position (e.g., coordinates) of sections 55a-55d of region 55, such that one or more of the ablation electrodes are placed in contact with the one or more sections of region 55. In the present example, ablation electrodes 44a, 44b, 44c and 44d are assigned for ablating sections 55a, 55b, 55c and 55d, respectively. During the ablation procedure, ablation electrodes 44a, 44b, 44c and 44d are placed in contact with sections 55a, 55b, 55c and 55d, respectively. For example, ablation electrode 44a is placed in contact with section 55a, and ablation electrode 44c is placed in contact with section 55c.

In some embodiments, distal-end assembly 40 is configured to sense a contact force between balloon 42 and the tissue of region 55. The contact force may be sensed between each ablation electrode (e.g., electrodes 44, and 44a-44d) and the tissue, or between the surface of distal-end assembly 40 and the tissue of region 55. The contact force sensing may be carried out using any suitable technique, such as but not limited to techniques described in U.S. Patent Nos. 8,357,152, 9,050,105, 10,791,950, and U.S. Patent Application Publication No. 2020/0206479 A1, whose disclosures are all incorporated herein by reference. Note that different sorts of contact force sensing techniques may be applied, *mutatis mutandis,* to different types of catheters (e.g., focal catheters, basket catheters, or balloon catheters).

In some embodiments, physician 30 (and/or processor 34) may define a threshold, also referred to herein as a force threshold, which is indicative of the required minimal level of contact force that must be applied, during ablation, by ablation electrodes (e.g., electrodes 44a-44d) to the tissue of one or more of sections 55a-55d of region 55. Note that in case the contact force is smaller than the force threshold, the tissue will not be ablated sufficiently.

In some embodiments, physician 30 (and/or processor 34) may define an energy level intended to be applied to at least one of, and typically all sections 55a-55d of region 55. Note that the applied energy depends on, *inter alia,* (i) the power and the duration of the ablation signal(s) applied to the respective section(s), and (ii) the contact force applied between the ablation electrode and the respective section of region 55. In the context of the present disclosure and in the claims, the term "duration" refers to a time interval in which an ablation electrode applies the ablation signal.

In other embodiments, one or more of the ablation electrodes of distal-end assembly 40 are placed in contact with the surface of one section of region 55. For example, ablation electrodes 55a and 55b are placed in contact with section 55a. Moreover, in case the amount of required ablation energy differs between different sections of region 55, the number of electrodes placed in contact with each section, may be different. For example, two electrodes 44 may be placed in contact with section 55a, and one electrode may be placed in contact with section 55d.

In some embodiments, the power and/or the duration of the applied ablation signal(s) may differ between the different sections of region 55. Each section may receive a typical power between about 3 Watts and 50 Watts and the typical duration of applying the power to the tissue may be between about 5 seconds and 60 seconds. For example, sections 55a and 55d may receive a power level of about 3 Watts for about 10 seconds, whereas sections 55b and 55c may receive the same power for about 20 seconds. Note that one or more of the variables described above (e.g., power, duration) are preconfigured by physician 30, e.g., using an operational recipe of system 20.

In other embodiments, the duration of the power applied to all sections 55a-55d may be similar, but the power of the ablation signal(s) applied to each section may be different. In yet other embodiments, any suitable combination of power and duration may be applied to each section of sections 55a-55d.

In some embodiments, before applying the ablation signal(s), processor 34 is configured check (e.g., based on position signals received by the interface that is described in Fig. 1 above) from position sensor 39) whether the respective electrode is placed in contact with the intended section of region 55. In case the ablation electrode is not positioned in contact with the intended section, processor 34 may prevent the application of the ablation signal(s) from a radiofrequency (RF) generator (not shown) of system 20, to the respective ablation electrode. For example, if ablation electrode 44a is not placed in contact with section 55a, processor 34 may present (e.g., on display 35) a message to physician 30, to adjust the position of electrode 44a. Moreover, after ablation electrode 44a has been placed in contact with section 55a, processor 34 checks whether or not the contact force applied between electrode 44a and section 55a is sufficiently high. In case the contact force is smaller than the force threshold, processor 34 may prevent the application of the ablation signal(s), and may present (e.g., on display 35) a message to physician 30, to adjust the contact force applied by electrode 44a to section 55a.

In some embodiments, the contact-force checking may be carried out after the position checking, in other embodiments, both checks may be carried out at the same time, and respective messages may be presented to physician 30 on display 30.

In some embodiments, after verifying that all ablation electrodes 44a-44d are properly placed in sufficiently high contact force with sections 55a-55d, respectively. Processor 34 is configured to control the RF generator to apply each ablation signal to each respective ablation electrode. Moreover, processor 34 is configured to monitor the power and duration of each ablation signal applied to each ablation electrode (e.g., electrodes 44a-44d), and to calculate the cumulative ablation energy applied, via the respective electrode, to the respective section of region 55.

In some embodiments, processor 34 is configured to hold a threshold value indicative of the energy level intended to be applied via each ablation electrode to each section of region 55. In some embodiments, in response to calculating that the amount of energy applied via a given electrode to a given section, exceeds the threshold indicative of the energy level, processor 34 is configured to control the RF generator to stop applying power to the respective ablation electrode. In other embodiments, processor 34 is configured to control a device configured to route the ablation signals to the intended ablation electrodes, to stop routing the ablation signal(s) to the respective ablation electrode. In other words, processor 34 is configured to terminating the ablation signal(s) applied to the respective ablation electrode.

For example, in case the cumulative ablation energy applied to electrode 44a exceeds (i.e., is not larger than) the threshold assigned to section 55a, processor 34 controls the RF generator and/or the routing device (and/or any other suitable mechanism configured to apply the ablation signal to electrode 44a), to stop applying the ablation signal to electrode 44a. Similarly, in case the cumulative ablation energy applied to electrode 44b does not exceed the threshold assigned to section 55b, processor 34 controls the RF generator and/or the routing device to continue applying the ablation signals to section 55b via electrode 44b.

In some embodiments, in response to identifying that the position and/or the contact force of a given electrode alters relative to the specified level when applying the ablation signal(s), processor 34 is configured to hold the application of the respective ablation signal(s) to the respective one or more ablation electrode(s).

In some embodiments, as soon as the cumulative amount of ablation energy exceeds the energy level threshold in all sections 55a-55d, processor 34 may present to physician 30, e.g., on display 35, a message indicative that the application of the ablation signal(s) has been completed, so that physician 30 may decide to: (i) extract distal-end assembly 40 out of heart 26, and conclude the ablation procedure, or (ii) move distal-end assembly 40 to another ablation site for conducting applying additional ablation signals to tissue at another location in heart 26 or in any other organ of patient 28.

Fig. 3 is a flow chart that schematically illustrates a method for applying ablation signals to tissue of region 55 using electrodes 44a-44d of distal-end assembly 40, in accordance with an embodiment of the present invention.

The method begins at a mark receiving step 100, with processor 34 receiving (e.g., from physician 30) and displaying a mark on map 27 indicative of a target tissue (e.g., region 55) intended to be ablated, as described in detail with respect to Figs. 1 and 2A and B above.

At an energy-level receiving step 102, processor 34 receives, for each section (e.g., of sections 55a-55d) of region 55, a specified energy level of the ablation signal(s) intended to be applied to the respective section during the ablation procedure, as described in detail with respect to Fig. 1 above.

At an electrode placement step 104, physician 30 moves distal-end assembly 40 toward region 55 and processor 34 receives, e.g., from position sensor 39 and/or from another position tracking system such as the ACL system described with respect to Figs. 2A and B above, position signals indicative of the position of distal-end assembly 40. Based on the position signals, processor is configured to calculate the position of each ablation electrode (e.g., electrodes 44a-44d) intended to be placed in contact with sections 55a-55d of region 55, as described in detail in Fig. 2 above.

At a position verification step 106, processor 34 checks whether or not each of the ablation electrodes is positioned at the respective intended position. For example, processor 34 checks whether electrodes 44a, 44b, 44c and 44d are placed in contact with sections 55a, 55b, 55c and 55d, respectively. At a position adjustment step 108, in response to identifying that one or more of electrodes 44a, 44b, 44c and 44d are not placed in contact with one or more of sections 55a, 55b, 55c and 55d, respectively, processor 34 may present a message to physician 30 (e.g., on display 35), to adjust the position of the one or more respective ablation electrode(s), as described in detail with respect to Figs. 2A and B above. In some embodiments, after adjusting the position of the one or more ablation electrodes, the method loops back to step 106 for verification, as described in detail in Fig. 2 above.

In other embodiments, step 108 is optional and may be eliminated from the method because processor 42 is configured to select from among the ablation electrodes of distal-end assembly 40, ablation electrodes that do fall on sections 55a, 55b, 55c and 55d of region 55 and have sufficient contact with sections 55a, 55b, 55c and 55d. For example, in case distal-end assembly 40 is moved, processor 34 is configured to select another set of ablation electrodes of distal-end assembly 40 that fall on sections 55a, 55b, 55c and 55d.

At a contact force verification step 110, processor 34 checks whether or not each of the ablation electrodes is placed in contact with the respective intended sections at a contact force larger than the force threshold, as described in detail with respect to Figs. 2A and B above. At a contact force adjustment step 112, in response to identifying that one or more of electrodes 44a, 44b, 44c and 44d are not placed in a sufficiently high contact force with one or more of sections 55a, 55b, 55c and 55d, respectively, processor 34 may present a message to physician 30 (e.g., on display 35), to adjust the contact force applied between the one or more respective ablation electrode (s) and the one or more respective sections of region 55, as described in detail in Fig. 2 above.

In some embodiments, after adjusting the contact force between one or more of the ablation electrodes and the tissue of the respective section of region 55, the method loops back to step 110 for verification, as described in detail with respect to Figs. 2A and B above.

In other embodiments, steps 110 and 112 are optional and may be removed from the method of Fig. 3 because, based on the position signals, processor 42 is configured to automatically select from among the ablation electrodes of distal-end assembly 40, ablation electrodes that do fall on sections 55a, 55b, 55c and 55d of region 55 and have sufficient contact with sections 55a, 55b, 55c and 55d. In such embodiments, no contact force measurement and/or adjustment is required.

At an ablation signal application step 114, in response to verifying that all ablation electrodes 44a-44d are positioned on and placed in sufficiently high contact with sections 55a-55d, respectively, processor 34 controls the RF generator to apply the predefined ablation signal(s) to sections 55a-55d using electrodes 44a-44d of distal-end assembly 40. Moreover, processor 34 holds thresholds indicative of the energy level intended to be applied to each section of sections 55a-55d, as described with respect to Figs. 2A and B above. Furthermore, processor 34 monitors the power and the duration of the applied ablation signals for calculating the cumulative amount of ablation energy applied to each section of sections 55a-55d, as described with respect to Figs. 2A and B above.

At an energy comparison step 116, processor 34 compares, for each section of region 55, whether or not the cumulative amount of applied ablation energy is larger than the threshold of energy level. In response to identifying, in a given ablation electrode and a respective section, that the cumulative amount of applied ablation energy is smaller than the threshold of the energy level, the method loops back to step 114 for continuing to apply the ablation signal(s) to the given ablation electrode, as described in detail with respect to Figs. 2A and B above.

In case the cumulative amount of applied ablation energy is larger than the threshold of energy level, the method proceeds to a decision step 118, in which processor 34 checks whether or not the cumulative amount of applied ablation energy is larger than the threshold of energy level in all sections 55a-55d. In response to identifying one or more sections in which the cumulative amount of applied ablation energy is smaller than the threshold of energy level, the method loops back to step 114 for continuing to apply the ablation signal(s) to the ablation electrode intended to apply the ablation signal(s) to the respective section.

In response to identifying that the cumulative amount of applied ablation energy is larger than the threshold of energy level in all sections 55a-55d, the method proceeds to a procedure ending step 120 that terminates the method. Note that when applying the ablation signal (s) to the selected ablation electrodes of distal-end assembly 40, selected ablation electrodes 44a, 44b, 44c and 44d, are configured to produce together a lesion having a shape that covers the predefined pattern of region 55.

In some embodiments, processor 34 may present to physician 30, e.g., on display 35, a message indicative that all sections 55a-55d have been ablated with the predefined amount of ablation energy, and physician 30 may extract catheter 22 out of heart 26, or proceed to the next ablation site, as described with respect to Figs. 2A and B above.

This particular sequence of the method of Fig. 3 is shown by way of example, in order to illustrate certain problems that are addressed by embodiments of the present invention and to demonstrate the application of these embodiments in enhancing the performance of such an ablation procedure. Embodiments of the present invention, however, are by no means limited to this specific sort of example sequence of steps, and the principles described herein may similarly be applied to other sorts of method for ablating tissue in heart 26 or in any other organ of patient 28.

For example, in other embodiments, steps 106 and 110 may be carried out at the same time or in a reversed order, steps 108 and 112 may be carried out at the same time or in a reversed order, and steps 116 and 118 may be carried out at the same time.

Although the embodiments described herein mainly address ablation of tissue in patient heart using a balloon catheter, the methods and systems described herein can also be used in other applications, such as in ablating the heart using any other suitable type of ablation catheter, and in ablation procedures carried out in other organs of a patient, and in ablation procedure using other sorts of RF ablation techniques and/or irreversible electroporation (IRE) .

It will thus be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

### Aspects of the invention:

1. A method for ablating a region of a patient organ using selected ablation electrodes of an expandable catheter, the method comprising:
   receiving: (i) a position of a target tissue intended to be ablated in an organ of a patient and having a predefined pattern, and (ii) an energy level of an ablation signal intended to be applied to the target tissue;
   selecting, in a catheter that is inserted into the organ and having an array of ablation electrodes, one or more selected ablation electrodes that, when applying the ablation signal, produce together a lesion having a shape that covers the predefined pattern; and
   in response to verifying that the one or more selected ablation electrodes are positioned on the target tissue, applying the ablation signal to the target tissue using the one or more selected ablation electrodes.
2. The method according to aspect 1, wherein applying the ablation signal to the target tissue comprises monitoring a cumulative energy of the ablation signal applied to the target tissue, and in response to detecting that the cumulative energy exceeds the energy level, terminating the ablation signal to the one or more selected ablation electrodes.
3. The method according to aspect 2, wherein monitoring the cumulative energy comprises monitoring an ablation power of the ablation signal and a time interval of applying the ablation signal to the target tissue using the one or more selected ablation electrodes.
4. The method according to aspect 1, wherein the target tissue comprises a first section at a first position and a second section at a second position different from the first position, and comprising receiving a position signal indicative of an additional position of the array of ablation electrodes, and calculating electrodes positions of the ablation electrodes of the array, respectively, and wherein the one or more selected ablation electrodes comprise at least a first ablation electrode for producing a first lesion that covers the first section and a second ablation electrode for producing a second lesion that covers the second section.
5. The method according to aspect 4, and comprising verifying that a contact force between the one or more selected ablation electrode and the target tissue is larger than a force threshold, wherein applying the ablation signal comprises verifying that the first ablation electrode is positioned on the first section and the second ablation electrode is positioned on the second section, and subsequently, verifying that the contact force between: (i) the first ablation electrode and the first section, and (ii) the second ablation electrode and the second section, is larger than the force threshold.
6. The method according to aspect 5, wherein when applying the ablation signal, in response to detecting that at least one of: (i) at least the first electrode is moved relative to the first section, and (ii) the contact force between at least the first ablation electrode and the first section is smaller than the force threshold, terminating the ablation signal to at least the first ablation electrode.
7. The method according to aspect 4, wherein receiving the first and second positions comprises receiving a region indicative of the target tissue, and receiving a first coordinate defining the first section, and a second coordinate defining the second section, and wherein selecting the first and second electrodes comprises selecting: (i) the first ablation electrode that falls on the first coordinate and (ii) the second ablation electrode that falls on the second coordinate.
8. The method according to aspect 7, wherein receiving the energy level comprises receiving a first energy level of a first ablation signal intended to be applied to the first section, and a second energy level of a second ablation signal intended to be applied to the second section, and wherein defining the first and second sections is based on at least one of: (i) a geometrical shape of the first and second sections, and (ii) the first and second energy levels.
9. The method according to aspect 7, wherein the first energy level differs from the second energy level.
10. The method according to aspect 1, wherein the catheter comprises an expandable distal-end assembly having the first and second ablation electrode and a third ablation electrode not selected by the processor, and wherein the ablation signal is not applied to the third ablation electrode.

## Claims

1. A system for ablating a region of a patient organ using selected ablation electrodes of an expandable catheter, the system comprising:
an interface, which is configured to receive: (i) a position of a target tissue intended to be ablated in an organ of a patient and having a predefined pattern, and (ii) an energy level of an ablation signal intended to be applied to the target tissue; and
a processor, which is configured to:
select, in a catheter that is inserted into the organ and having an array of ablation electrodes, one or more selected ablation electrodes that, when applying the ablation signal, produce together a lesion having a shape that covers the predefined pattern; and
in response to verifying the one or more selected ablation electrodes are positioned on the target tissue, control a generator to apply the ablation signal to the target tissue using the one or more selected ablation electrodes.

2. The system according to claim 1, wherein the processor is configured to: (i) monitor a cumulative energy of the ablation signal applied to the target tissue, and (ii) in response to detecting that the cumulative energy exceeds the energy level, terminate the ablation signal to the one or more selected ablation electrodes.

3. The system according to claim 2, wherein the processor is configured to monitor an ablation power of the ablation signal and a time interval of applying the ablation signal to the target tissue using the one or more selected ablation electrodes.

4. The system according to claim 1, wherein the target tissue comprises a first section at a first position and a second section at a second position different from the first position, wherein the interface is configured to receive a position signal indicative of an additional position of the array of ablation electrodes, wherein the processor is configured to calculate electrodes positions of the ablation electrodes of the array, respectively, and wherein the one or more selected ablation electrodes comprise at least a first ablation electrode configured for producing a first lesion that covers the first section and a second ablation electrode configured for producing a second lesion that that covers the second section.

5. The system according to claim 4, wherein the processor is configured to verify that the first ablation electrode is positioned on the first section and the second ablation electrode is positioned on the second section, and subsequently, to verify that a contact force between: (i) the first ablation electrode and the first section, and (ii) the second ablation electrode and the second section, is larger than a force threshold.

6. The system according to claim 5, wherein, in response to detecting that at least one of: (i) at least the first electrode is moved relative to the first section, and (ii) the contact force between at least the first ablation electrode and the first section is smaller than the force threshold, the processor is configured to terminate the ablation signal to at least the first ablation electrode.

7. The system according to claim 4, wherein the interface is configured to receive: a region indicative of the target tissue, and (ii) a first coordinate defining the first section, and a second coordinate defining the second section, the processor is configured to select: (i) the first ablation electrode that falls on the first coordinate and (ii) the second ablation electrode that falls on the second coordinate.

8. The system according to claim 7, wherein the interface is configured to receive a first energy level of a first ablation signal intended to be applied to the first section, and a second energy level of a second ablation signal intended to be applied to the second section, and wherein the processor is configured to define the first and second sections based on at least one of: (i) a geometrical shape of the first and second sections, and (ii) the first and second energy levels.

9. The system according to claim 7, wherein the first energy level differs from the second energy level.

10. The system according to claim 1, wherein the catheter comprises an expandable distal-end assembly having the first and second ablation electrode and a third ablation electrode not selected by the processor, and wherein the processor is configured to control the generator not to apply the ablation signal to the third ablation electrode.
